# EUROPÄISCHE PATENTSCHRIFT

(11) **EP 1 322 588 B1**
(45) Veröffentlichungstag und Bekanntmachung des Hinweises auf die Patenterteilung: **29.06.2016**
(21) Anmeldenummer: 01971467.4
(22) Anmeldetag: 05.10.2001
(51) Int. Cl.: C07C 67/03, C07C 63/24, C07C 69/52, C11C 3/04, C11C 3/10

(54) **VERFAHREN ZUR HERSTELLUNG VON FETTSÄUREALKYLESTERN**
METHOD FOR PRODUCING FATTY ACID ALKYL ESTERS
PROCEDE DE PRODUCTION D'ESTERS ALKYLIQUES D'ACIDES GRAS

(30) Priorität: 05.10.2000 AT 20001699
(43) Veröffentlichungstag der Anmeldung: 02.07.2003
(73) Patentinhaber: BDI - BioEnergy International AG, 8074 Grambach/Graz (AT); Koncar, Michael, 8501 Lieboch (AT); Mittelbach, Martin, 8010 Graz (AT)
(72) Erfinder: KONCAR, Michael, A-8501 Lieboch (AT); MITTELBACH, Martin, A-8010 Graz (AT); GÖSSLER, Helmut, A-8042 Graz (AT); HAMMER, Wilhelm, A-8074 Grambach (AT)
(74) Vertreter: Schwarz, Albin
(86) Internationale Anmeldenummer: PCT/AT2001/000320
(87) Internationale Veröffentlichungsnummer: WO 2002/028811

(56) Entgegenhaltungen:
- EP-A- 0 708 813
- GB-A- 612 667
- US-A- 2 521 742
- US-A- 5 424 466
- LAGO R C A ET AL: "EXTRACTION AND TRANSESTERIFICATION OF VEGETABLE OILS WITH ETHANOL" OLEAGINEUX, PARIS, FR, Bd. 40, Nr. 3, 1985, Seiten 147-151, XP002025504 ISSN: 0030-2082 in der Anmeldung erwähnt

## Beschreibung

Die vorliegende Erfindung betrifft ein Verfahren zur Herstellung von Fettsäurealkylestern durch alkalisch katalysierte Umesterung eines Gemisches von Triglyceriden und Fettsäuren, wobei aus einem Reaktionsgemisch, in welchem die Umesterung durchgeführt wird, eine Fettsäurealkylester enthaltende Esterphase und eine Fettsäuren enthaltende Glycerinphase gebildet werden, die voneinander getrennt werden, und die Fettsäuren aus der Glycerinphase durch Behandeln mit einer Säure abgetrennt werden, wobei eine Fettsäuren enthaltende Fettsäurephase gebildet wird, welche Fettsäuren mit einem Alkohol verestert werden.

Unter Triglyceriden werden für die Zwecke der vorliegenden Beschreibung und Patentansprüche Ester von höheren, gesättigten und/oder ungesättigten Fettsäuren mit Glycerin verstanden. Derartige Ester sind z.B. Fette pflanzlichen oder tierischen Ursprungs und auch Altspeiseöle und Abfallfette. Viele Fette natürlichen Ursprungs enthalten aber auch noch freie Fettsäuren in einem mehr oder minder großen Ausmaß. Diese Fette sind somit ein Gemisch aus Triglyceriden und freien Fettsäuren, wobei der Hauptbestandteil dieses Gemisches in der Regel die Triglyceride sind.

Unter Umestenung ist die Alkoholyse von Triglyceriden zu verstehen, also die Umsetzung mit Alkoholen, insbesondere Methanol und Ethanol, wobei über die Zwischenprodukte Di-und Monoglyceride die Monoester der Fettsäuren sowie Glycerin entstehen.

Fettsäureester, insbesondere die Methylester, sind wichtige Zwischenprodukte in der Oleochemie. Allein in Europa werden jährlich 200.000 Tonnen Pflanzenölmethylester als Rohstoffe vor allem für Tenside hergestellt. Daneben gewinnt der Fettsäuremethylester als Dieselersatzkraftstoff immer mehr an Bedeutung.

Als Katalysatoren für die Umesterung können basische Katalysatoren (Alkalihydroxide, -alkoholate, -oxide, -carbonate, Anionenaustauscher), saure Katalysatoren (Mineralsäuren, p-Toluolsulfonsäure, Bortrifluorid, Kationenaustauscher) und Enzyme (Lipasen) verwendet werden. Bevorzugt werden heute im Reaktionsgemisch lösliche Katalysatoren verwendet. Diese bilden ein homogenes Gemisch und gewährleisten schnelle Umsatzraten und milde Reaktionsbedingungen. Die am häufigsten verwendeten homogenen Katalysatoren sind Natrium- und Kaliumhydroxid sowie Natriummethylat, welche in Alkohol gelöst dem Pflanzenöl zugemischt werden. Ein derartiges Verfahren ist aus der AT-B 386 222 bekannt. Die saure Katalyse erfordert höhere Reaktionstemperaturen und -drücke und eine aufwendigere Reaktionsführung. Eine saure Umesterung ist aus der FR-A - 85 02340 bekannt.

Die Umesterung mit basischer Katalyse wird ohne Verwendung eines Lösungsmittels durchgeführt. Die Reaktion beginnt mit einem Zweiphasensystem aus Triglycerid und Alkohol, mit zunehmendem Reaktionsfortschritt und Bildung von Ester entsteht aber eine homogene Phase, welche durch Bildung und Ausscheidung von Glycerin wiederum zweiphasig wird.

Bei der Alkoholyse von Triglyceriden zur Herstellung von Estern der Fettsäuren mit einwertigen Alkoholen fällt als Nebenprodukt eine glycerinreiche Phase an. Diese Phase enthält ferner Fettsäuren, Fettsäuresalze und Fettsäureester. Um diese Fettsäureverbindungen aus der Glycerinphase abzutrennen, wird sie in der Regel mit Säuren behandelt. Durch diese Behandlung werden die Fettsäuren aus den Fettsäuresalzen freigesetzt. Die Fettsäuren sowie die Fettsäureester selbst sind nicht mit Glycerin mischbar und setzen sich daher als eigene Phase von der Glycerinphase ab. Diese Phase wird als Fettsäurephase bezeichnet.

Ein Verfahren der eingangs erwähnten Art ist der EP-A - 0 708 813 zu entnehmen. Dieses vorbekannte Verfahren verwertet die Fettsäurephase, indem die in dieser Phase enthaltenen Fettsäuren mit einem Alkohol verestert und die erhaltenen Fettsäurealkylester einem anderen Reaktionsgemisch, in welchem eine Umesterung gerade vorgenommen wird, zugegeben werden.

Wie bereits erwähnt, enthalten Fette natürlichen Ursprungs auch freie Fettsäuren in einem unterschiedlichen Ausmaß. Je höher dieser Anteil an freien Fettsäuren ist, desto weniger Triglycerid steht als Rohstoff für die Umesterung zur Verfügung. Umgekehrt kann die Ausbeute an Fettsäurealkylester erhöht werden, wenn die freien Fettsäuren in einem getrennten Schritt verestert werden, wie dies in der oben genannten EP-A - 0 708 813 der Fall ist.

Aus Oleagineux, Vol. 40, Nr. 3, Seiten 148-151 (1985) ist bekannt, die in der Fettsäurephase enthaltenen freien Fettsäuren mit ethanolhältiger Miscella unter Verwendung von Schwefelsäure als Katalysator zum Ethylester zu verestern, die Schwefelsäure mit CaO zu neutralisieren, das gebildete Kalciumsulfat abzufiltrieren, den erhaltenen Ester mit Umesterungskatalysator zu vermischen und mit dieser Mischung eine ölreiche Miscella umzuestern. Miscella wird bei der Extraktion des öl- bzw. fetthaltigen Rohstoffs erhalten und ist somit eine Mischung aus Extraktionsmittel und umzuesterndem Triglycerid. Dieses Verfahren ist sehr aufwendig und eignet sich nicht gut, ölhaltige Rohstoffe, welche freie Fettsäuren enthalten, umzuestern.

Die vorliegende Erfindung setzt hier an und stellt sich zur Aufgabe, ein Verfahren der eingangs beschriebenen Art zur Verfügung zu stellen, gemäß welchem die Fettsäurephase unbehandelt, d.h. ohne Reinigung und Entfernung von Schwefelsäure, weiterverarbeitet werden kann und bei welchem auch Rohstoffe mit einem höheren Gehalt an freien Fettsäuren umgeestert werden können.

Das erfindungsgemäße Verfahren ist dadurch gekennzeichnet, daß die Fettsäurephase mit einem weiteren Gemisch von Triglyceriden und Fettsäuren vermischt und die in der erhaltenen Mischung enthaltenen Fettsäuren mit einem Alkohol verestert werden, wobei ein Veresterungsgemisch erhalten wird, welches Triglyceride und Fettsäurealkylester enthält, welches Veresterungsgemisch zur Bildung von weiteren Fettsäurealkylestern mit Alkohol umgeestert wird, wobei das mit der Fettsäurephase vermischte Gemisch von Triglyceriden und Fettsäuren einen Fettsäuregehalt von mindestens 5 Gew.-% aufweist.

Gemäß dem erfindungsgemäßen Verfahren werden somit die im umzuesternden Fett bzw. Öl (d.h. Gemisch aus Triglyceriden und Fettsäuren) enthaltenen Fettsäuren vor der eigentlichen Umesterung mit einem Alkohol verestert, wobei diese Veresterung in Gegenwart der Fettsäurephase einer vorhergegangenen Umesterung durchgeführt wird, sodaß sowohl die im umzuesternden Fett enthaltenen Fettsäuren als auch die in der zugegebenen Fettsäurephase enthaltenen Fettsäuren verestert werden.

Die Fettsäurephase aus der vorhergegangenen Umesterung braucht nicht gereinigt zu werden und kann als solche, d.h. samt überschüssigem Methanol, dem Fett zugegeben werden.

Enthält das Fett einen sehr hohen Anteil an freien Fettsäuren, kann das nach der ersten Veresterung erhaltene Veresterungsgemisch vor der Umesterung noch mindestens ein weiteres Mal mit Alkohol verestert werden. Auf diese Weise läßt sich der Gehalt an freien Fettsäuren schrittweise herabsetzen, sodaß eine Ausbeute an Fettsäurealkylestern von 100 % möglich wird.

Eine bevorzugte Ausführungsform des erfindungsgemäßen Verfahrens ist daher dadurch gekennzeichnet, daß das mit der Fettsäurephase vermischte Gemisch von Triglyceriden und Fettsäuren einen Fettsäuregehalt von mindestens 10 Gew.-%. aufweist.

Die Veresterung wird bevorzugt unter saurer Katalyse durchgeführt.

Im erfindungsgemäßen Verfahren werden als Alkohol zur Umesterung und zur Veresterung insbesondere Methanol oder Ethanol eingesetzt.

Eine beispielhafte Ausführungsform der Erfindung wird mit dem nachfolgenden Beispiel noch näher beschrieben.

### Beispiel

### 1. Veresterung

Zunächst wurden 1.000 g gebrauchtes Speiseöl mit einem Gehalt an freien Fettsäuren von 7,15 % zur Veresterung mit 100 g Methanol und 7,0 g H₂SO₄ (98 %) versetzt und 2 Stunden unter Rückfluß gekocht. Der Ansatz wurde in einen Scheidetrichter übergeführt und in eine wässrige Phase (51,6 g) und in eine Ölphase (1045,0 g) aufgetrennt.

### 2. Umesterung

Die Ölphase wurde einer zweistufigen alkalischen Umesterung gemäß dem in der AT-B 386.222 beschriebenen Verfahren unterzogen. Dabei wurde die Ölphase mit insgesamt 192,2 g Methanol und 12,19 g KOH vermischt und die entstandene Glycerinphase nach jeder Stufe abgetrennt. Die entstandene Esterphase (917,8 g) wurde in einem Rotavapor entmethanolisiert (40,8 g), sodaß 877,0 g Fettsäuremethylester erhalten wurden.

### 3. Bildung der Fettsäurephase

Die beiden aus der Umesterung gewonnenen Glycerinphasen (328,1 g) wurden durch Zugabe von 12,0 g H₂SO₄ auf einen pH-Wert von ca. 3 gebracht. Durch diese Ansäuerung entstanden aus den in der Glycerinphase vorhandenen Kaliseifen freie Fettsäuren und Kaliumsalz, das als feste Phase vorlag. Das entstandene Gemisch wurde filtriert und das Filtrat in einen Scheidetrichter übergeführt. Nach der Phasentrennung lagen folgende Mengen vor: Fettsäurephase 103,5 g, Glycerinphase 200,2 g, Filterrückstand 32,8 g.

### 4. Veresterung

Die so gewonnene Fettsäurephase (103,5 g) wurde mit 896,5 g gebrauchtem Speiseöl mit einem Gehalt an freien Fettsäuren von 7,15 % vermischt und zur Veresterung mit 100 g Methanol und 7,0 g H₂SO₄ (98 %) 2 Stunden lang unter Rückfluß gekocht. Der Ansatz wurde in einen Schütteltrichter überführt und in eine wässrige Phase (59,2 g) und in eine Ölphase (1.047,8 g) aufgetrennt. Der Gehalt an freien Fettsäuren im Öl betrug 0,98 %.

### 5. Umesterung

Die Ölphase wurde wiederum einer zweistufigen alkalischen Umesterung gemäß AT-B - 386.222 unterzogen. Dabei wurde die Ölphase mit insgesamt 190,8 g Methanol und 12,11 g KOH velinischt und die entstandene Glycerinphase nach jeder Stufe abgetrennt. Die entstandene Esterphase (1.016,4 g) wurde in einem Rotavapor entmethanolisiert (56,3 g), wobei als reine Esterphase 907,1 g verblieben.

### 6. Bildung der Festtsäurephase

Die Glycerinphasen aus der Umesterung (282,1 g) wurden zusammengeführt und durch Zugabe von 10,1 g H₂SO₄ auf einen pH-Wert von ca. 3 gebracht. Das entstandene Gemisch wurde filtriert und das Filtrat in einem Scheidetrichter übergeführt. Nach der Phasentrennung lagen folgende Mengen vor: Fettsäurephase 97,2 g, Glycerinphase 161,2 g, Filterrückstand 33,8 g.

Die erhaltene Fettsäurephase kann neuerlich mit gebrauchtem Speiseöl vermischt und zur weiteren Herstellung von Methylester verwendet werden.

## Patentansprüche

1. Verfahren zur Herstellung von Fettsäurealkylestern durch alkalish Katalysierte, eines Gemisches von Triglyceriden und Fettsäuren, wobei aus einem Reaktionsgemisch, in welchem die Umesterung durchgeführt wird, eine Fettsäurealkylester enthaltende Esterphase und eine Fettsäuren enthaltende Glycerinphase gebildet werden, die voneinander getrennt werden, und die Fettsäuren aus der Glycerinphase durch Behandelen mit einer säure abetrennt werden, wobei eine fettsauren enthaltende Fettsaurephase gebildet wird, welche Fettsäuren mit einem Alkohol verestert werden,
**dadurch gekennzeichnet, daß**
die Fettsäurephase mit einem weiteren Gemisch von Triglyceriden und Fettsäuren vermischt und die in der erhaltenen Mischung enthaltenen Fettsäuren mit einem Alkohol verestert werden, wobei ein Veresterungsgemisch erhalten wird, welches Triglyceride und Fettsäurealkylester enthält, welches Veresterungsgemisch zur Bildung von weiteren Fettsäurealkylestern mit Alkohol umgeestert wird, wobei das mit der Fettsäurephase vermischte Gemisch von Triglyceriden und Fettsäuren einen Fettsäuregehalt von mindestens 5 Gew.-% aufweist.

2. Verfahren nach Anspruch 1, **dadurch gekennzeichnet, daß** das Veresterungsgemisch vor der Umesterung ein weiteres Mal mit Alkohol verestert wird.

3. Verfahren nach Anspruch 1, **dadurch gekennzeichnet, daß** das mit der Fettsäurephase vermischte Gemisch von Triglyceriden und Fettsäuren einen Fettsäuregehalt von mindestens 10 Gew.-% aufweist.

4. Verfahren nach einem der Ansprüche bis 3, **dadurch gekennzeichnet, daß** die Veresterung unter saurer Katalyse durchgeführt wird.

5. Verfahren nach einem der Ansprüche 1 bis 8, **dadurch gekennzeichnet, daß** als Alkohol zur Umesterung und zur Veresterung Methanol oder Ethanol eingesetzt wird.

## Claims

1. A method for the preparation of fatty acid alkyl esters by alkali-catalyzed transesterification of a mixture of triglycerides and fatty acids, wherein from a reaction mixture, in which the transesterification is carried out, an ester phase containing fatty acid alkyl esters and a glycerol phase containing fatty acids are formed, which are separated from each other, and the fatty acids are separated from the glycerol phase by treatment with an acid, whereby a fatty acid phase containing fatty acids is formed, which fatty acids are esterified with an alcohol,
**characterized in that**
the fatty acid phase is mixed with a further mixture of triglycerides and fatty acids and the fatty acids contained in the obtained mixture are esterified with an alcohol, whereby an esterification mixture containing triglycerides and fatty acid alkyl esters is obtained, which esterification mixture is transesterified with alcohol so as to form further fatty acid alkyl esters, wherein the mixture of triglycerides and fatty acids mixed with the fatty acid phase exhibits a fatty acid content of at least 5% by weight.

2. A method according to claim 1, **characterized in that**, prior to the transesterification, the esterification mixture is esterified with alcohol for another time.

3. A method according to claim 1, **characterized in that** the mixture of triglycerides and fatty acids mixed with the fatty acid phase exhibits a fatty acid content of at least 10% by weight.

4. A method according to anyone of claims 1 to 3, **characterized in that** the esterification is carried out under acidic catalysis.

5. A method according to anyone of claims 1 to 4, **characterized in that** methanol or ethanol is employed as an alcohol for the transesterification and the esterification.

## Revendications

1. Procédé de production d'esters alkyliques d'acides gras par transestérification catalysée par voie alcaline d'un mélange de triglycérides et d'acides gras, en formant à partir d'un mélange réactionnel, dans lequel la transestérification est réalisée, une phase ester contenant des esters alkyliques d'acides gras et une phase glycérine contenant des acides gras, lesquelles sont séparées l'une de l'autre, et les acides gras sont séparés de la phase glycérine par traitement avec un acide, ce qui permet de former une phase acides gras contenant des acides gras, lesdits acides gras étant estérifiés avec un alcool,
**caractérisé en ce que**
la phase acides gras est mélangée avec un autre mélange de triglycérides et d'acides gras et les acides gras contenus dans le mélange obtenu sont estérifiés avec un alcool, ce qui permet d'obtenir un mélange d'estérification qui contient des triglycérides et des esters alkyliques d'acides gras, ledit mélange d'estérification étant transestérifié avec un alcool pour former d'autres esters alkyliques d'acides gras, le mélange de triglycérides et d'acides gras, mélangé avec la phase acides gras, présentant une teneur en acides gras d'au moins 5 % en poids.

2. Procédé selon la revendication 1, **caractérisé en ce que** le mélange d'estérification est estérifié une autre fois avec un alcool avant la transestérification.

3. Procédé selon la revendication 1, **caractérisé en ce que** le mélange de triglycérides et d'acides gras, mélangé avec la phase acides gras, présente une teneur en acides gras d'au moins 10 % en poids.

4. Procédé selon l'une des revendications 1 à 3,
**caractérisé en ce que** l'estérification est réalisée sous catalyse acide.

5. Procédé selon l'une des revendications 1 à 4,
**caractérisé en ce que** du méthanol ou de l'éthanol est mis en oeuvre comme alcool pour la transestérification et pour l'estérification.
